(19) Europäisches Patentamt
European Patent Office
Office européen des brevets

(11) Veröffentlichungsnummer: **0 206 149**
**B1**

(12) # EUROPÄISCHE PATENTSCHRIFT

(45) Veröffentlichungstag der Patentschrift: **23.08.89**

(51) Int. Cl.⁴: **C 07 C 121/75**, C 07 C 120/00

(21) Anmeldenummer: **86108037.2**

(22) Anmeldetag: **12.06.86**

(54) **Verfahren zur Herstellung bestimmter Enantiomerenpaare von Permethrinsäure-alpha-cyano-3-phenoxy-4-fluor-benzyl-ester.**

(30) Priorität: **25.06.85 DE 3522629**

(43) Veröffentlichungstag der Anmeldung: **30.12.86 Patentblatt 86/52**

(45) Bekanntmachung des Hinweises auf die Patenterteilung: **23.08.89 Patentblatt 89/34**

(84) Benannte Vertragsstaaten: **AT BE CH DE FR GB IT LI NL SE**

(56) Entgegenhaltungen:
**EP-A-0 107 296**
**EP-A-0 150 006**
**EP-A-0 171 894**
**GB-A-2 064 528**

(73) Patentinhaber: **BAYER AG, D-5090 Leverkusen 1 Bayerwerk (DE)**

(72) Erfinder: **Fuchs, Rainer, Dr., Roeberstrasse 8, D-5600 Wuppertal 1 (DE)**
Erfinder: **Wittig, Andreas, Dr., Am Friedenshain 33, D-5600 Wuppertal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 206 149 B1

LIBER, STOCKHOLM 1989

**Beschreibung**

Die vorliegende Erfindung betrifft ein Verfahren zur Herstellung bestimmter Enantiomerenpaare von Permethrinsäure-α-cyano-3-phenoxy-4-fluor-benzylester ausgehend vom Gemisch aller sterischen und optischen Isomeren.

Es ist bekannt, daß Enantiomere von Verbindungen mit einem aciden Wasserstoffatom an einem asymmetrischen C-Atom durch Behandeln mit Basen epimerisiert werden können. Die bei der Reaktion mit Basen entstehenden Carbanionen gehen ständig rasch in ihre denkbaren enantiomeren Formen über. Sie passieren dabei kurzzeitig den ebenen Zustand (P. Sykes: Reaktionsaufklärung - Methoden und Kriterien der organischen Reaktionsmechanistik: Verlag Chemie 1973, S. 133 und D.J. Cram: Fundamentals in Carbanion Chemistry, S. 85 - 105, Academic Press New York (1965).

Dieser Fall wird z. B. auch bei der leichten basenkatalysierten Epimerisierung von optisch aktiver Mandelsäurenitril der Formel

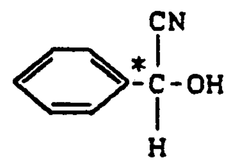

und des entsprechenden Methylethers der Formel

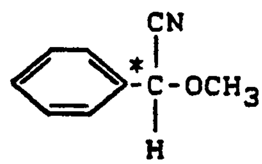

zu den razemischen Verbindungen beobachtet, (Smith: J. Chem. Soc. 1935, S. 194 und Smith: Ber, 64 (1931) S. 427.)

Je nach Löslichkeit der Gleichgewichtspartner eines Epimerisierungsgleichgewichtes im labilen Diastereomeren kann das Gleichgewicht sehr stark oder vollständig auf eine Seite verschoben werden, wenn ein Teil auskristallisiert. Dieser Fall wird als "second order asymmetric transformation" bezeichnet. (K. Mislow, Introduction to Stereochemistry, W.C. Benjamin Inc. New York, Amsterdam 1966, Seite 122 oben.)

Dieser Effekt kann jedoch nur zur praktischen Anwendung gebracht werden, wenn es gelingt, ein Lösungsmittel zu finden, in dem ein Stereoisomer und/oder sein Spiegelbild leichter und das andere Stereoisomer und/oder sein Spiegelbild schwerer löslich ist.

Eine solche Reaktion ist beispielsweise bereits mit dem optisch aktiven 2,2-Dimethyl-3R-(2,2-dichlorvinyl)-cyclopropan-1R-carbonsäure-α-cyano-(αRS)-3-phenoxybenzylester bekannt geworden (DE-OS-2 718 039). Als epimerisierende Basen werden Ammoniak und Amine eingesetzt. Als Lösungsmittel werden Acetonitril sowie niedere Alkanole eingesetzt. Dabei wird jedoch vom Ester eines bestimmten Enantiomeren der Carbonsäure (1R3R) ausgegangen.

Es fehlt jeder Hinweis darauf, daß dieses Verfahren auch eingesetzt werden kann, um aus dem razemischen Gemisch alle 8 Stereoisomeren der obigen Verbindung eine Abtrennung von gewissen Stereoisomeren durch Epimerisierung der anderen zu erreichen.

Des weiteren ist aus DE-OS-2 903 057 bekannt, daß auch die 4 stereoisomeren α-Cyano-(αR,S)-3-phenoxybenzylester einer razemischen Carbonsäure durch Behandlung mit Basen am α-Kohlenstoffatom neben der Cyanogruppe epimerisiert werden können und aus geeigneten Lösungsmitteln ein einzelnes Enantiomerenpaar auskristallisiert werden kann. Auch hier werden als geeignete Lösungsmittel niedere Alkohole, insbesondere Methanol, genannt. Als Base wird wäßriger Ammoniak eingesetzt.

In ähnlicher Weise verläuft nach EP-OS-22 382 ein Verfahren zur Umwandlung des Stereoisomerengemisches der 4 cis-Isomeren von Permethrinsäure-α-cyano-3-phenoxy-benzylester in ein reines Enantiomerenpaar durch Auskristallisieren des schwerer löslichen Enantiomerenpaares in einem geeigneten Lösungsmittel, durch anschließendes Epimerisieren des in Lösung verbleibenden anderen Enantiomerenpaares mit einer Base und erneutes Auskristallisieren des schwerer löslichen Enantiomerenpaares. Kristallisieren und Epimerisieren werden dabei in getrennten Stufen vorgenommen. Als geeignetes Lösungsmittel werden dabei Kohlenwasserstoffe, insbesondere Hexan, offenbart. Als Basen werden Amine, insbesondere Triethylamin, eingesetzt.

Aus DE-OS-3 115 881 ist ein weiteres Verfahren zur Umwandlung des Stereoisomerengemisches aller 4 cis-Isomeren von α-cyan-3-phenoxybenzyl-3-(2,2-dichlorvinyl)-2,2-dimethyl-cyclopropancarbonsäureester in ein einzelnes Enantiomerenpaar bekannt geworden. Dabei wird als Lösungsmittel und gleichzeitig als Base ein organisches Amin verwendet. Als gut geeignet wird dabei Triethylamin und Diisopropylamin beschrieben. Als ungeeignet wird Tri-n-propylamin und n-Butylmethylamin genannt. Auch bei diesem Verfahren wird jedoch mit einem sterisch einheitlichen razemischen Säureteil (cis-Isomeren) gearbeitet. Auch hier fehlt jeder Hinweis

darauf, ob eine Abtrennung einzelner Stereoisomeren auch aus dem Gemisch aller 8 denkbaren Stereoisomeren möglich ist.

Es ist nicht möglich, Vorhersagen darüber zu machen, welche Lösungsmittel sich zur Trennung von Enantiomeren oder Diastereomeren der Enantiomerenpaare eignen.

Es ist daher erforderlich, für jede einzelne Verbindung und bei cis-trans Isomeren auch für jedes einzelne cis- und/oder trans-Stereoisomerenpaar, ein geeignetes Trennsystem zu entwickeln. Erfahrungen aus prinzipiell ähnlich gelagerten Fällen lassen sich nur gelegentlich und dann in nicht vorhersehbarer Weise übertragen.

Insbesondere geht es aus der angegebenen Literatur nicht hervor, ob und mit welchen Lösungsmitteln es möglich ist, die gewünschten cis- und trans-Diastereomeren gleichzeitig abzutrennen.

2,2-Dimethyl-3-dichlorvinyl-cyclopropan-carbonsäure-(Permethrinsäure)-α-cyano-3'-phenoxy-4'-fluorbenzylester besitzt die Strukturformel I

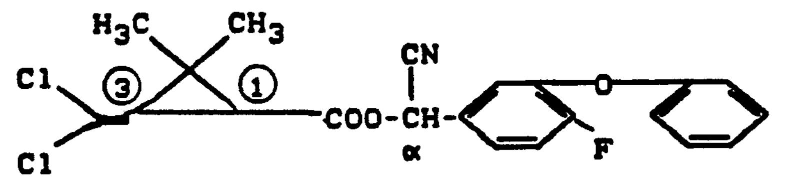

I

Die Verbindung besitzt drei asymmetrische Zentren Fig, Fig und α. Sie liegt damit in folgenden Enantiomerenpaaren vor:

| | |
|---|---|
| a: 1R-3R-αR + 1S-3S-αS<br>b: 1R-3R-αS + 1S-3S-αR | 1,3 cis |
| c: 1R-3S-αR + 1S-3R-αS<br>d: 1R-35-αS + 1S-3R-αR | 1,3 trans |

Besonders wirksam gegen zahlreiche wirtschaftlich interessante Schädlinge sind die Enantiomerenpaare b und d.

Bei der technischen Herstellung der Verbindung der Formel I sind die Verhältnisse der Enantiomerenpaare a - d nur in einem gewissen engen Rahmen variabel. Bei einer typischen technisch hergestellten Verbindung der Formel liegen die Enantiomerenpaare a - d z. B. in folgendem Verhältnis vor (bezogen auf 100 %)

a = 24,5 %
b = 17,5 %
c = 34,5 %
d = 23,5 %.

Es sollte ein Verfahren gefunden werden, mit dem sich das Verhältnis der Enantiomerenpaare a - d im Gemisch aller Enantiomeren zugunsten der Enantiomerenpaare b und d verändert.

Es wurde gefunden, daß man ein Gemisch aller 8 Stereoisomeren der Verbindung Permethrinsäure-α-cyano-3-phenoxy-4-fluor-benzylester in die Enantiomerenpaare

b) 1R-3R-αS + 1S-3S-αR und
d) 1R-3S-αS + 1S-3R-αR

dadurch überführen kann, daß man dieses Gemisch aller Stereoisomeren in einem Alkanol mit 2 - 4 C-Atomen löst, als Base ein sekundäres oder tertiäres Amin mit jeweils 2 - 6 C-Atomen je Alkylteil zusetzt und aus der erhaltenen Lösung das Gemisch der Enantiomerenpaare b und d auskristallisiert, wobei das Verhältnis der gemeinsam auskristallisierten Enantiomerenpaare b/d dem cis/trans Verhältnis der eingesetzten Enantiomerenpaare (a+b)/(c+d) entspricht.

Es war überraschend, daß es für diesen Vorgang nicht erforderlich war, von einem in Säureteil sterisch einheitlichen Ester auszugehen, sondern daß eine Umwandlung des technisch anfallenden Gemisches aus allen 8 Stereoisomeren der cis- und trans-Reihe in ein Gemisch, bestehend aus nur noch im wesentlichen 4 cis- und trans-Stereoisomeren gelang.

Das erfindungsgemäße Verfahren wird in einem Alkanol mit 2 - 4 C-Atomen als Lösungsmittel durchgeführt. Bevorzugt wird Isopropanol verwendet.

Als Base werden sekundäre oder tertiäre Alkylamine mit jeweils 2 - 6 C-Atomen je Alkylteil verwendet. Bevorzugt werden Di-iso-Butylamin und Tri-n-Butylamin verwendet.

Das Mischungsverhältnis zwischen Alkanol und Amin (in Gewichtsteilen) kann in einem Bereich von Alkanol/Amin = 1000/1 bis 1/10 variieren. Bevorzugt wird ein Mischungsverhältnis Alkanol/Amin von 100/0,5 bis 100/20.

Das Mischungsverhältnis der eingesetzten technischen Ware der Strukturformel I mit dem verwendeten Alkanol (in Gewichtsteilen) kann in einem Bereich von I/Alkanol = 10/1 bis 1/10 variieren. Bevorzugt ist ein Bereich von 3/1 bis 1/3.

Die Alkanole und Amine werden im wesentlichen wasserfrei eingesetzt. Das technische cis/trans-Ausgangsmaterial wird in der Mischung aus Alkanol und Aminbase bei 40 - 80° C, bevorzugt zwischen 50 und 70° C, gelöst. Man kühlt anschließend die Lösung auf -25 bis +30° C ab. Die Kristallisation kann durch Zugabe einiger Kleinkristalle der Enantiomerenpaare b + d beschleunigt werden. Jedoch erfolgt die Kristallisation auch schon spontan. Die Isolierung der Enantiomerenpaare b + d erfolgt in üblicher Weise z. B. durch Filtration oder Zentrifugieren.

Die nachfolgenden Beispiele erläutern die Erfindung, ohne eine Beschränkung hinsichtlich ihrer Reichweite anzugeben.

Für die Beispiele wurde ein technisches Produkt folgender Zusammensetzung benutzt:

| | |
|---|---|
| Isomerenzusammensetzung | Ia = 25,2 % |
| (bezogen auf 100 %) | Ib = 19,2 % |
| | Ic = 32,2 % |
| | Id = 23,3 % |

Wirkstoffgehalt = 92 %
(Ia+b+c+d)

**Beispiel 1**

100 g eines technischen cis/trans-Gemisches aller 8 Stereoisomeren von α-Cyano-3-phenoxy-4-fluorbenzyl-3-(2,2-dichlorvinyl-2,2-dimethyl-cyclopropancarbonsäureester werden unter Erwärmen auf 50°C in 100 g Isopropanol gelöst. Nach dem Abkühlen auf 20° C werden zu dieser Mischung 4 g Diisobutylamin gegeben. Anschließend wird bei 20 - 23° C gerührt. Hierbei setzt meist eine spontane Kristallisation ein, die durch Zugabe einiger Impfkristalle von Ib und Id beschleunigt werden kann. Nach einer Rührzeit von 9 Tagen bei 20 - 23° C wird die Reaktionsmischung auf 5° C abgekühlt und die entstandenen Kristalle abgesaugt. Die Kristalle werden 2 x mit je 50 ml eiskaltem Isopropanol gewaschen, trockengesaugt und an der Luft getrocknet.

Man erhält 82,2 g (87,8 % der Theorie) eines farblosen kristallienen Produkts mit einem Schmelzpunkt von 82 - 89° C und folgender durch HPLC ermittelter Isomerenzusammensetzung (bezogen auf 100 %):

Ia = 0,8 %, Ib = 38,8 %, Ic = 1,9 %, Id = 58,5 %.

**Beispiel 2**

100 g des technischen cis/trans Gemisches aller 8 Stereoisomeren von α-Cyano-3-phenoxy-4-fluorbenzyl-3-(2,2-dichlorvinyl)-2,2-dimethyl-cyclopropancarbonsäureester werden wie in Beispiel 1 beschrieben mit den in Tabelle 1 aufgeführten Alkanolen und Aminen umgesetzt. Die Ergebnisse sind in Tabelle 1 zusammengefaßt.

**Tabelle 1**

| Alkanol + Amin | Menge in g | Rührzeit in Tagen | Ausbeute an kristallinem Produkt in % d.Th. | Isomerenzusammensetzung Ia | Ib | Ic | Id |
|---|---|---|---|---|---|---|---|
| Methanol | 100 | 9 | 0 | - | - | - | - |
| Di-iso-Butylamin | 4 | | es tritt Zersetzung ein | | | | |
| n-Propanol | 100 | 9 | 64 | 0,5 | 31,8 | 1,9 | 65,8 |
| Di-iso-Butylamin | 4 | | | | | | |
| n-Butanol | 100 | 9 | 51,3 | 0,4 | 16,1 | 2,2 | 81,0 |
| Di-iso-Butylamin | 4 | | | | | | |
| sec.-Butanol | 100 | 9 | 49 | - | - | - | - |
| Di-iso-Butylamin | 4 | | | | | | |
| tert.-Butanol | 100 | 9 | 40 | - | - | - | - |
| Di-iso-Butylamin | 4 | | | | | | |
| Ethanol | 100 | 7 | 55 | 0,24 | 15,8 | 1,4 | 82,6 |
| Di-iso-Butylamin | 4 | | | | | | |

## Patentansprüche

1. Verfahren zur Herstellung des Gemisches folgender Enantiomerenpaare der Verbindung Permethrinsäure-α-cyan-3-phenoxy-4-fluorbenzylester:
b) 1R-3R-αS + 1S-3S-αR und
d) 1R-3S-αS + 1S-3R-αR,
dadurch gekennzeichnet, daß man ein Gemisch aller 8 Stereoisomeren der Verbindung Permethrinsäure-α-cyano-3-phenoxy-4-fluorbenzylester in einem Alkohol mit 2 bis 4 Kohlenstoffatomen löst, ein sekundäres oder tertiäres Amin mit jeweils 2 - 6 Kohlenstoffatomen je Alkylteil als Base zusetzt und aus der erhaltenen Lösung das Gemisch der Enantiomerenpaare b) und d) auskristallisiert.

2. Verfahren gemäß Anspruch 1, dadurch gekennzeichnet, daß man als Base Di-iso-Butylamin und/oder Tri-n-butylamin einsetzt.

3. Verfahren gemäß Anspruch 1, dadurch gekennzeichnet, daß man als Alkohol mit 2 bis 4 Kohlenstoffatomen Isopropanol einsetzt.

4. Verfahren gemäß Anspruch 1 bis 3, dadurch gekennzeichnet, daß das Mischungsverhältnis Alkohol/Amin in Gewichtsteilen von 1000/1 bis 1/10 beträgt.

5. Verfahren gemäß Anspruch 1 bis 4, dadurch gekennzeichnet, daß das Mischungsverhältnis Alkohol/Amin in Gewichtsteilen von 100/0,5 bis 100/20 beträgt.

## Claims

1. Process for the preparation of the mixture of the following pairs of enantiomers of the compound α-cyano-3-phenoxy-4-fluorobenzyl permethrate:
b) 1R-3R-αS + 1S-3S-αR and
d) 1R-3S-αS + 1S-3R-αR,
characterized in that a mixture of all 8 stereoisomers of the compound α-cyano-3-phenoxy-4-fluorobenzyl permethrate is dissolved in an alcohol having 2 to 4 carbon atoms, a secondary or tertiary amine having, in each case, 2 - 6 carbon atoms in each alkyl moiety is added as the base, and the mixture of the pairs of enantiomers b) and d) is crystallized out of the resulting solution.

2. Process according to Claim 1, characterized in that di-iso-butylamine and/or tri-n-butylamine is used as the base.

3. Process according to Claim 1, characterized in that isopropanol is used as the alcohol having 2 to 4 carbon atoms.

4. Process according to Claim 1 to 3, characterized in that the ratio of mixing of alcohol/amine is, in parts by weight, from 1,000/1 to 1/10.

5. Process according to Claim 1 to 4, characterized in that the ratio of mixing alcohol/amine is, in parts by weight, from 100/0.5 to 100/20.

## Revendications

1. Procédé de préparation du mélange des paires d'énantiomères du composé ester α-cyano-3-phénoxy-4-fluorobenzylique d'acide perméthrique:
b) 1R-3R-αS + 1S-3S-αR et
d) 1R-3S-αS + 1S-3R-αR,
caractérisé en ce qu'on dissout un mélange de tous les 8 stéréoisomères du composé ester α-cyano-3-phénoxy-4-fluorobenzylique d'acide perméthrique dans un alcool ayant 2 à 4 atomes de carbone, ajoute en tant que base une amine secondaire ou tertiaire avec, suivant le cas, 2 à 6 atomes de carbone par portion alkyle et sépare par cristallisation à partir de la solution obtenue le mélange des paires d'énantiomères b) et d).

2. Procédé selon la revendication 1, caractérisé en ce qu'on met en oeuvre en tant que base, de la diisobutylamine et/ou de la tri-n-butylamine.

3. Procédé selon la revendication 1, caractérisé en ce qu'on met en oeuvre de l'isopropanol en tant qu'alcool comportant 2 à 4 atomes de carbone.

4. Procédé selon les revendications 1 à 3, caractérisé en ce que le rapport de mélange alcool/amine en parties en poids s'étend de 1000/1 à 1/10.

5. Procédé selon les revendications 1 à 4, caractérisé en ce que le rapport de mélange alcool/amine en parties en poids s'étend de 100/0,5 à 100/20.